(19) **Europäisches Patentamt**
**European Patent Office**
**Office européen des brevets**

(11) **EP 4 215 903 B1**

(12) **EUROPEAN PATENT SPECIFICATION**

(45) Date of publication and mention
of the grant of the patent:
**06.11.2024   Bulletin 2024/45**

(51) International Patent Classification (IPC):
*G01N 21/27* (2006.01)      *C12Q 1/6844* (2018.01)
*C12Q 1/6853* (2018.01)

(21) Application number: **22171425.6**

(22) Date of filing: **03.05.2022**

(52) Cooperative Patent Classification (CPC):
**G01N 21/272**

(54) **TEST APPARATUS AND METHOD FOR DETERMINING WHETHER INFECTION IS PRESENT USING RATIO OF COLOR CHANGE OF REAGENT**

TESTGERÄT UND VERFAHREN ZUR BESTIMMUNG DES VORHANDENSEINS EINER INFEKTION UNTER VERWENDUNG DES VERHÄLTNISSES DER FARBÄNDERUNG DES REAGENS

APPAREIL ET PROCÉDÉ DE TEST POUR DÉTERMINER SI UNE INFECTION EST PRÉSENTE À L'AIDE DE RAPPORT DE CHANGEMENT DE COULEUR DE RÉACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **21.01.2022   KR 20220009053**

(43) Date of publication of application:
**26.07.2023   Bulletin 2023/30**

(73) Proprietor: **1Drop Inc.**
**Gyeonggi-do 13217 (KR)**

(72) Inventors:
 • **BAEK, So Hyeon**
  **12248 Gyeonggi-do (KR)**
 • **HONG, Seong Soo**
  **12779 Gyeonggi-do (KR)**
 • **PARK, Jeong Ho**
  **13011 Gyeonggi-do (KR)**
 • **BAG, Jeong In**
  **14071 Gyeonggi-do (KR)**
 • **RHEE, Joo Won**
  **13562 Gyeonggi-do (KR)**

(74) Representative: **advotec.**
**Patent- und Rechtsanwaltspartnerschaft**
**Tappe mbB**
**Widenmayerstraße 4**
**80538 München (DE)**

(56) References cited:
**WO-A1-2021/025043      CN-A- 111 763 768**
**US-A1- 2005 003 374      US-A1- 2016 348 159**
**US-A1- 2018 016 627      US-A1- 2020 332 340**

 • **CHANG WEN-HSIN ET AL: "Rapid isolation and detection of aquaculture pathogens in an integrated microfluidic system using loop-mediated isothermal amplification", SENSORS AND ACTUATORS B: CHEMICAL, ELSEVIER BV, NL, vol. 180, 26 December 2011 (2011-12-26), pages 96 - 106, XP029002626, ISSN: 0925-4005, DOI: 10.1016/J.SNB.2011.12.054**
 • **FENG JUNLI ET AL: "Detection ofListeria monocytogenesbased on combined aptamers magnetic capture and loop-mediated isothermal amplification", FOOD CONTROL, vol. 85, 26 October 2017 (2017-10-26) - 26 October 2017 (2017-10-26), pages 443 - 452, XP085291531, ISSN: 0956-7135, DOI: 10.1016/J.FOODCONT.2017.10.027**

**Description**

CROSS-REFERENCE TO RELATED APPLICATION

This application claims priority to and the benefit of Korean Patent Application No. 2022-0009053, filed on January 21, 2022.

BACKGROUND

**1. Field of the Invention**

The present invention relates to molecular diagnosis, and more particularly, to a technology capable of determining whether an infection is present using a change in color of a reagent.

**2. Discussion of Related Art**

The content described in this section merely provides background information for embodiments described in this specification and does not necessarily constitute the related art.

Since coronavirus disease 2019 (COVID-19) was first identified in Wuhan, China in December 2019, the number of confirmed cases and deaths worldwide has continued to increase. COVID-19 was initially known as an infectious respiratory disease of unknown cause, but has been identified as a new type of coronavirus by the World Health Organization (WHO). As COVID-19 spreads not only in Korea but also in Europe, the United States, etc., the WHO declared a pandemic on March 11, 2020 which is the highest risk level among the infectious disease warning levels, for COVID-19 and warned of its seriousness.

As of 2022, for the diagnosis of COVID-19 in Korea, a method of separating a virus from a sample or detecting a specific viral gene by using diagnostic test criteria is used regardless of clinical features. A genetic test used in the above is real-time reverse transcription polymerase chain reaction (RT-PCR). It is known that the real-time RT-PCR has an advantage in that it is an accurate test with close to 100% sensitivity and specificity, but has a disadvantage in that it requires professional personnel to collect the sample and takes a long time to obtain a test result.

Recently, fatigue due to the prolonged COVID-19 pandemic has accumulated and the daily average of new confirmed cases has rapidly increased to 600 to 700 so that a need for the introduction of "self-diagnosis kits" for COVID-19 is increasing. In the United States and many European countries, as the number of confirmed cases is already on the rise again, self-diagnosis kits are being recommended to the public to use as a "prevention option."

However, the self-diagnosis kit has a disadvantage in that it is less sensitive than a genetic test method (e.g., polymerase chain reaction (PCR)), in which medical personnel or a test expert collects a sample from the nasopharynx deep in the nose. It has been reported that although performance of the self-diagnosis kit is degraded due to a difference in measurement principles, a difference in sensitivity may appear depending on a method of collecting a sample. Researchers at the University of Birmingham in UK reported in the academic journal Public Library of Science (PLOS) Biology on April 29 that the accuracy of the PCR test varies considerably depending on the technique used to collect the sample with a swab, with 79% for skilled medical personnel and 58% for the general public, and differences in sensitivity and specificity may occur due to differences in the techniques of collecting the sample with a swab.

Accordingly, there is a need for a method of determining whether an infection is present even when an amount of a sample collected is small.

[Document of Related Art]

[Non-Patent Document]

(Non-Patent Document 0001) Jack Ferguson, Steven Dunn, Angus Best., "Validation testing to determine the sensitivity of lateral flow testing for asymptomatic SARS-CoV-2 detection in low prevalence settings: Testing frequency and public health messaging is key." PLOS Biology, (April 29, 2021)

Further prior art can be found in US 2018 / 0 016 627 A1 and in Chang, Wen-Hsin, et al. "Rapid isolation and detection of aquaculture pathogens in an integrated microfluidic system using loop-mediated isothermal amplification." Sensors and Actuators B: Chemical 180 (2013): 96-106.

SUMMARY OF THE INVENTION

The present invention is directed to providing a test apparatus and a test method, which are capable of determining whether an infection is present through molecular diagnosis.

Objects of the present invention are not limited to the above-described object and other objects that are not described will be clearly understood by those skilled in the art from the following descriptions.

According to an aspect of the present invention, there is provided a test apparatus as set out in the appended set of claims.

The test apparatus may further include a heating unit configured to heat the test tube and the control tube according to a control signal output from the controller.

The first time point may be a time point adjacent to a time point at which the controller terminates the heating operation of the heating unit, and the second time point may be a time point when a preset time has elapsed since the controller terminated the heating operation of the heating unit.

The test apparatus may further include a temperature sensor configured to output a temperature value of the control tube, the test tube, or the heating unit. In this case, the second time point may be a time point when the temperature value output from the temperature sensor reaches a preset temperature value after the controller terminates heating operation of the heating unit.

The controller may determine whether the sample is infected using the following equation:

$$K = (T_2 - T_1) / (C_2 - C_1)$$

- K denotes an infection determination value,
- $T_2$ denotes a value output from the second spectrum sensor at the second time point,
- $T_1$ denotes a value output from the second spectrum sensor at the first time point,
- $C_2$ denotes a value output from the first spectrum sensor at the second time point, and
- Ci denotes a value output from the first spectrum sensor at the first time point.

When the infection determination value (K) is greater than or equal to a preset reference value, the controller may determine that a result of determining whether the sample is infected is positive.

When the value of the signal output from the second spectrum sensor at the second time point is less than or equal to a preset primary infection determination reference value, the controller may determine whether the sample is infected using the test spectrum difference value compared to the control spectrum difference value.

When the value of the signal output from the first spectrum sensor at the second time point is greater than or equal to a preset measurement invalidity determination reference value and when the value of the signal output from the second spectrum sensor at the second time point is less than or equal to a preset primary infection determination reference value, the controller may determine whether the sample is infected using the test spectrum difference value compared to the control spectrum difference value.

The test apparatus may further include a communication unit configured to transmit the value of the signal output from the first spectrum sensor, the value of the signal output from the second spectrum sensor, or information on whether the sample is infected as determined by the controller.

The test apparatus may be one component of a diagnostic system including a test apparatus, and an external terminal configured to receive data transmitted from the test apparatus and display information on whether a sample is infected.

According to an aspect of the present invention, there is provided a test method as set out in the appended set of claims.

The first time point may be a time point relatively earlier than the second time point.

The first time point and the second time point may have an interval which is greater than or equal to a preset time.

The operation (c) may include an operation of determining, by the processor, whether the sample is infected using the following equation:

$$K = (T_2 - T_1) / (C_2 - C_1)$$

- K denotes an infection determination value,
- $T_2$ denotes a value output from the second spectrum sensor at the second time point,
- $T_1$ denotes a value output from the second spectrum sensor at the first time point,
- $C_2$ denotes a value output from the first spectrum sensor at the second time point, and
- Ci denotes a value output from the first spectrum sensor at the first time point.

The operation (c) may include, when the infection determination value (K) is greater than or equal to a preset reference value, an operation of determining, by the processor, that a result of determining whether the sample is infected is positive.

The operation (c) may include, when the value of the signal output from the second spectrum sensor at the second time point is less than or equal to a preset primary infection determination reference value, an operation of determining, by the processor, whether the sample is infected using the test spectrum difference value compared to the control spectrum difference value.

The operation (c) may include, when the value of the signal output from the first spectrum sensor at the second time point is greater than or equal to a preset measurement invalidity determination reference value, and when the value of the signal output from the second spectrum sensor at the second time point is less than or equal to a preset primary infection determination reference value, an operation of determining, by the processor, whether the sample is infected using the test spectrum difference value compared to the control spectrum difference value.

The test method according to the present invention may be implemented in the form of a computer program written to cause the test apparatus to perform each of the operations of the test method on a computer and recorded on a computer-readable recording medium.

The test method according to the present invention may be implemented using a terminal including a processor configured to cause the test apparatus to perform each of the operations of the test method, and a communication unit configured to receive, from a test apparatus, a value of a signal output from the first spectrum sensor and a value of a signal output from the second spectrum sensor.

The terminal may be one component of a diagnostic system including a terminal, and a test apparatus including the first spectrum sensor and the second spectrum sensor.

Other specific details of the present invention are included in the detailed description and accompanying drawings.

BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:

FIG 1 is a reference diagram for helping with understanding of a diagnostic system according to the present invention;
FIG 2 is a schematic block diagram of a configuration of a test apparatus according to the present invention;
FIG 3 illustrates exemplary views of tubes whose color is changed;
FIG 4 is an exemplary table for interpreting results according to the color of tubes;
FIG 5 illustrates an example of changes in absorbance over time of a control tube and a test tube with respect to a sample determined as negative;
FIG 6 illustrates an example of changes in absorbance over time of a control tube and a test tube with respect to a sample determined as positive;
FIG 7 illustrates an example of changes in absorbance over time of a control tube and a test tube with respect to a sample, which is positive but collected in insufficient quantities; and
FIG 8 is a flowchart of a test method according to an embodiment of the present invention.

DETAILED DESCRIPTION OF EXEMPLARY EMBODIMENTS

Advantages and features of the present invention and methods of achieving the same will be clearly understood with reference to the accompanying drawings and embodiments described in detail below. However, the present invention is not limited to the embodiments disclosed below but may be implemented in various different forms. The embodiments are provided in order to fully describe the present embodiments and fully explain the scope of the present invention to those skilled in the art. The scope of the present invention is only defined by the appended claims.

Like reference numerals indicate like components throughout the specification and the term "and/or" includes each and all combinations of one or more referents. It should be understood that, although the terms "first," "second," etc. may be used herein to describe various components, these components are not limited by these terms. The terms are only used to distinguish one component from another component.

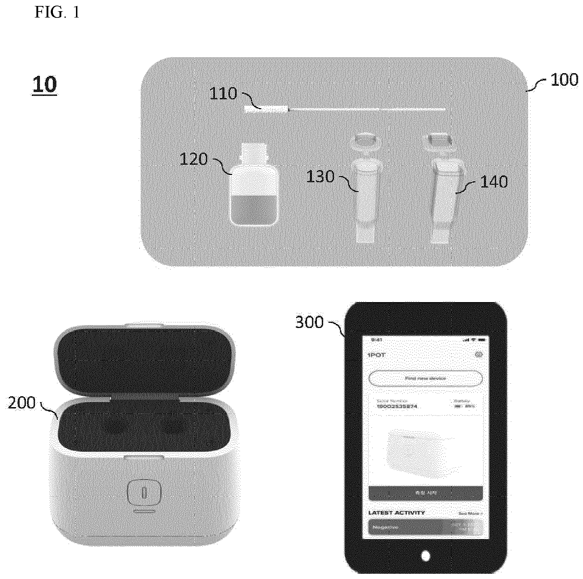

FIG. 1 is a reference diagram for helping with understanding of a diagnostic system according to the present invention.

Referring to FIG 1, a diagnostic system 10 according to the present invention may include a test apparatus 200 and an external terminal 300.

The test apparatus 200 is an apparatus capable of performing a test on a sample collected from a respiratory system. The test apparatus 200 may be a molecular diagnostic apparatus or an antigen diagnostic apparatus. In this specification, as an example of the test apparatus 200, an amplifier capable of performing a test on a sample using a molecular diagnostic method is provided. The molecular diagnostic method refers to a method of checking whether a disease is infected by extracting deoxyribonucleic acid (DNA) or ribonucleic acid (RNA) from a sample (e.g., saliva, blood, etc.) of a person infected with a virus or bacteria and then amplifying the DNA or RNA using gene amplification technology. Depending on the gene amplification method, there are various methods such as polymerase chain reaction (PCR), reverse transcription polymerase chain reaction (RT-PCR), loop-mediated isothermal amplification (LAMP), and the like. In this specification, an example of the test apparatus 200 for determining whether an infection is present by qualitatively determining a novel coronavirus (severe acute respiratory syndrome coronavirus 2 (SARS-CoV-2)) gene (spike (S) gene, nucleocapsid (N) gene) using LAMP with a sample collected from the nasopharynx or nasal cavity of a patient suspected of being infected with the novel coronavirus (SARS-CoV-2) will be described. However, a target for which the test apparatus 200 determines whether an infection is present, or a molecular diagnosis method performed by the test apparatus 200 is not limited by the above example.

In order to perform a test on a sample using the test apparatus 200 according to the present invention, a test kit may be required. The test kit refers to a tool, a reagent, or a consumable, which is necessary for the test, and may be sealed and provided in a pouch 100.

Referring to FIG 1, a swab 110, a sample tube 120, a test tube 130, and a control tube 140 may be identified. The swab 110, the sample tube 120, the test tube 130, and the control tube 140 may be manufactured for one-time use by one subject. Therefore, the swab 110, the sample tube 120, the test tube 130, and the control tube 140 may be provided as new components for each test to perform additional and repeated tests. Further, the swab 110, the sample tube 120, the test tube 130, and the control tube 140 may be manufactured in a sterile state and may be sealed in the pouch 100 and distributed.

The swab 110 is a tool (sampling tool) for a subject to collect a sample. A length and thickness of the swab 110 may have values suitable for collecting the sample.

The sample tube 120 may include a solution for nucleic acid extraction. Specifically, the solution for nucleic acid extraction may include a buffer solution, a nonionic surfactant, a kosmotropic salt, and an indicator.

In the solution for nucleic acid extraction, the buffer solution may be a Tris-buffer solution. Specifically, the buffer solution may be Tris-HCl of pH 8 to pH 9 or pH 8.3 to pH 8.6. A concentration of the buffer solution may range from 1.0 mM to 2 mM.

In the solution for nucleic acid extraction, the nonionic surfactant may be a Tween-type surfactant. Specifically, the nonionic surfactant may be Tween 20. A concentration of the nonionic surfactant may range from 0.05 vol% to 0.5 vol% or 0.07 vol% to 0.2 vol%.

In the solution for nucleic acid extraction, the kosmotropic salt may include at least one selected from the group consisting of ammonium sulfate, potassium chloride, and magnesium sulfate. Specifically, the kosmotropic salt may include all of the ammonium sulfate, the potassium chloride, and the magnesium sulfate. In this case, a concentration of the ammonium sulfate may range from 5 mM to 20 mM or 7.5 mM to 15 mM. Further, a concentration of the potassium chloride may range from 20 mM to 100 mM or 40 mM to 60 mM. Furthermore, a concentration of the magnesium sulfate may range from 3 mM to 10 mM or 4 mM to 8 mM.

In the solution for nucleic acid extraction, the indicator may include at least one selected from the group consisting of cresol red and phenol red, but the present invention is not limited thereto, and an appropriate indicator may be used according to the purpose of use.

Further, the solution for nucleic acid extraction may further include an additive such as guanidine. When the solution for nucleic acid extraction includes guanidine, a concentration of the guanidine may range from 10 mM to 80 mM or 30 mM to 50 mM.

The test tube 130 and the control tube 140 may include primers for LAMP. The primer that is contained in the test tube 130 and the primer that is contained in the control tube 140 are different primers. The primer contained in the test tube 130 is a primer capable of amplifying the nucleic acid of a virus that is a target for checking whether an infection is present, and the primer contained in the control tube 140 is a primer capable of amplifying a nucleic acid that can be collected from a general person.

Further, the test tube 130 and the control tube 140 may further include a 0.1 to 1 vM primer mix, a 32 KU/T Bst DNA polymerase, a 12.5 mU/T ribonuclease (RNase) inhibitor, 20 to 200 uM deoxynucleotide triphosphates (dNTPs), 20 vg/T bovine serum albumin (BSA), 1.2 vg/T trehalose, a 1.6 vg sample buffer, or the like.

The test apparatus 200 may perform a test on a sample by itself without external interference, or may communicate with the external terminal 300 to perform a test on a sample. The test apparatus 200 may have a structure of which a lid is opened or closed, and may have a space in which the test tube 130 and the control tube 140 are accommodated when the lid is opened. According to an embodiment of the present invention, the test tube 130 and the control tube 140 may have different shapes (e.g., a cylindrical shape and a quadrangular container shape) for easy distinction, and a space may be formed inside the test apparatus 200 to correspond to the shapes of the test tube 130 and the control tube 140.

Meanwhile, since it is possible for the test apparatus 200 to perform a test on the sample using LAMP, a configuration required for LAMP may be included.

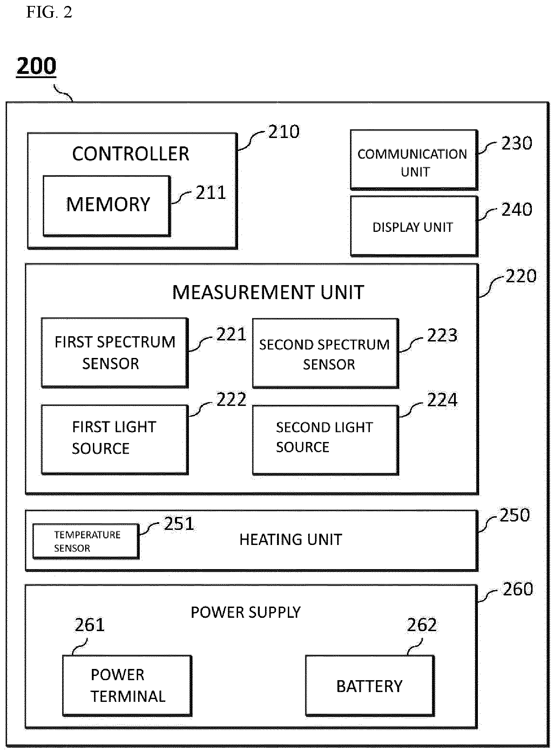

FIG. 2 is a schematic block diagram of a configuration of the test apparatus according to the present invention.

Referring to FIG 2, the test apparatus 200 according to the present invention may include a controller 210, a measurement unit 220, a communication unit 230, a display unit 240, a heating unit 250, and a power supply 260.

The controller 210 may control a process necessary for performing a test on a sample using LAMP. When the controller 210 can output a signal for controlling each component included in the test apparatus 200, the controller 210 may receive the signal from each component and perform necessary processes such as calculation, storage, processing, etc.

The measurement unit 220 may serve to measure the test tube 130 and the control tube 140 according to a procedure for performing a test on a sample. As will be described below in more detail, depending on the presence or absence of the nucleic acid, as the nucleic acid is amplified, the colors of the solutions which are contained in the test tube 130 and the control tube 140 may be changed. The measurement unit 220 may measure the colors of the solutions which are contained in the test tube 130 and the control tube 140 and output the measured colors as signals to the controller 210. To this end, the measurement unit 220 may include spectrum sensors 221 and 223 and/or light sources 222 and 224.

The communication unit 230 may serve to perform data transmission and reception between the test apparatus 200 and the external terminal 300. The communication unit 230 may perform wired communication and/or wireless communication, and may transmit and receive data according to a preset communication protocol. Preferably, the communication unit 230 may be a short range communication module for short range communication. The short range communication module may support short range communication using at least one of Bluetooth™, radio frequency identification (RFID), the Infrared Data Association (IrDA), ultra-wideband (UWB), ZigBee, near-field communication (NFC), Wi-Fi, Wi-Fi Direct, and wireless Universal Serial Bus (USB).

The display unit 240 may serve to display status information and operation information about the test apparatus 200. For example, the display unit 240 may include two light-emitting diodes (LEDs) installed on an external front surface of the test apparatus 200. A first LED may display a communication connection state between the test apparatus 200 and the external terminal 300. A second LED may display a charging state and a normal operation state of the test apparatus 200.

The heating unit 250 may serve to heat the test tube 130 and the control tube 140. The heating unit 250 may be configured as a coil to receive power from the power supply 260 in response to a control signal of the controller 210 and convert the power into thermal energy. The coil may have a length and resistance sufficient to heat the test tube 130 and the control tube 140 to a preset temperature. According to an embodiment of the present invention, the heating unit 250 may further include a temperature sensor 251. The controller 210 may output a signal for controlling the operation of the heating unit 250 according to a temperature signal output from the temperature sensor 251.

The power supply 260 may serve to supply power required for the operation of the components included in the test apparatus 200 according to the present invention. To this end, the power supply 260 may include a power terminal 261 and a battery 262. The battery 262 may be one of a primary battery and a secondary battery, and preferably, the battery 262 is a secondary battery. The power terminal 261 is an interface for connection with an external power supply source (e.g., a commercial power grid, a rechargeable battery, etc.), and, for example, the power terminal may be configured as a USB input/output terminal. The power supply 260 may charge the battery 262 with power applied through the power terminal 261. In order to perform a test on a sample, the power charged in the battery 262 may be used, or the power may be directly received from the power terminal 261 and used.

The external terminal 300 according to the present invention may be a communication terminal capable of performing data transmission and reception, for example, a smartphone, a laptop computer, a digital broadcasting terminal, a personal digital assistant (PDA), a portable multimedia player (PMP), a navigation system, a slate personal computer (PC), a tablet PC, an Ultrabook, a wearable device (e.g., a watch-type terminal (smartwatch), a glass-type terminal (smart glass), a head mounted display (HMD)), or the like. In the external terminal 300 according to the present invention, an application program necessary for performing a test on a sample may be installed.

An example of a method of diagnosing whether a viral infection is present using the test apparatus 200 according to the present invention will be described. In this case, it is assumed that the battery included in the test apparatus 200 is fully charged and an application program necessary for performing a test on a sample is installed in the external terminal 300.

First, power of the test apparatus 200 may be turned on by pressing a (power) button positioned on a front surface of the test apparatus 200. In addition, the test apparatus 200 and the external terminal 300 may be connected by executing the application program installed in the external terminal 300.

Next, a subject may collect a sample by pushing the swab 110 up to the nasopharynx inside his or her nose. The swab 110, which completed sample collection, may be put into the sample tube 120 and the sample tube 120 may be sufficiently shaken about 20 to 30 times, and thus the sample may be mixed in the sample tube 120.

Next, a preset amount (e.g., 50 ul) of the sample mixed by sufficiently shaking the sample tube 120 may be added to the test tube 130 and the control tube 140. The test tube 130 and the control tube 140 are mounted at their places in the test apparatus 200, and the lid of the test apparatus 200 is closed. When the lid of the test apparatus 200 is closed, the test apparatus 200 may be driven for a preset time at a preset temperature. In this case, the remaining time among a total time may be displayed on a display screen of the external terminal 300.

When the preset time has elapsed, the display screen of the external terminal 300 may be transitioned to a driving completion screen, and a determination result may be displayed on the display screen. In this case, the measurement unit 220 of the test apparatus 200 may output a signal for a change in color of the test tube 130 and the control tube 140. The signal output from the measurement unit 220 may be stored in the memory 211 of the controller 210.

The controller 210 may obtain a result of determining whether an infection is present by using data on the change in the color of the test tube 130 and the control tube 140. Alternatively, the controller 210 may transmit data on the signal stored in the memory 211 to the external terminal 300 through the communication unit 230. The external terminal 300 may obtain a result of determining whether an infection is present by using the data on the change in the color of the test tube 130 and the control tube 140 and display the result on the display screen.

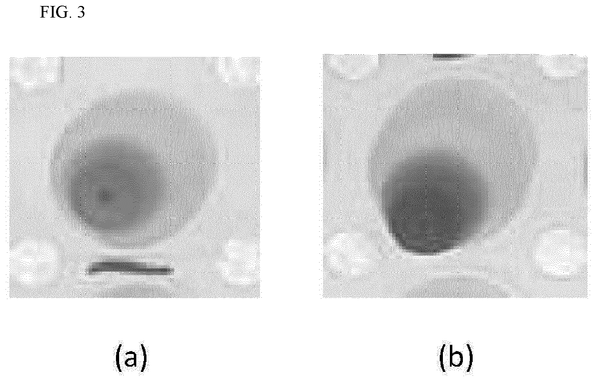

FIG 3 illustrates exemplary views of tubes whose color is changed.

Referring to FIG 3A, it can be seen that a solution contained in a tube is yellow, and referring to FIG 3B, it can be seen that a solution contained in a tube is red. Each of the test tube 130 and the control tube 140 may contain a reagent whose color is changed according to pH. In the example shown in FIG 3, the "yellow" may indicate "positive" meaning that detection target nucleic acid is contained therein, and the "red" may indicate "negative" meaning that the detection target nucleic acid is not contained therein.

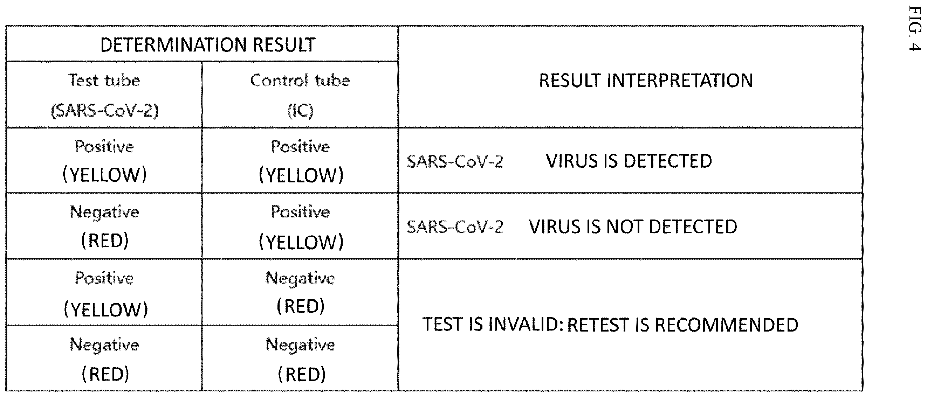

FIG. 4 is an exemplary table for interpreting results according to colors of tubes.

Referring to the table shown in FIG 4, when a control tube is measured to be yellow, this means that a sample is well extracted and the LAMP reaction is also well performed, indicating that the measurement result is reliable. On the other hand, when the control tube is measured to be red, this means that the sample is not well extracted or the LAMP reaction is well performed, indicating that the measurement result is unreliable. In this case, the test is invalid and thus a retest may be recommended.

Meanwhile, when the control tube is measured to be yellow and the test tube is measured to be yellow, this means that coronavirus disease 2019 (COVID-19) virus has been detected. That is, it may be determined that the subject is infected with the COVID-19 virus. On the other hand, when the control tube is measured to be yellow and the test tube is measured to be red, this means that no COVID-19 virus has been detected. That is, it may be determined that the subject is not infected with the COVID-19 virus. It is obvious that the color may be variously changed according to the types of the reagents contained in the test tube 130 and the control tube 140.

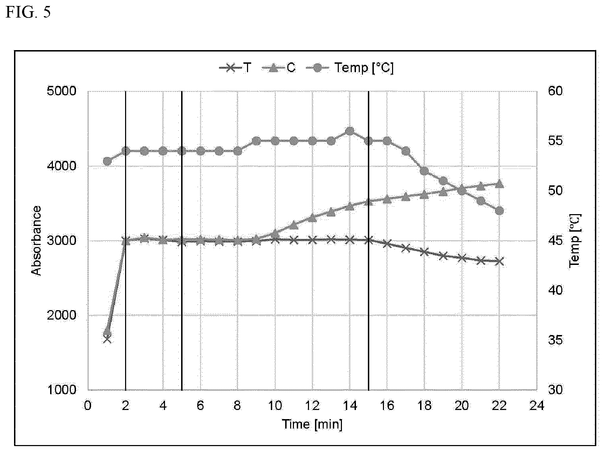

FIG 5 illustrates an example of changes in absorbance over time of a control tube and a test tube with respect to a sample determined as negative.

Referring to FIG 5, a horizontal axis represents time (min), a left vertical axis represents absorbance, and a right vertical axis represents temperature (Temp). According to an embodiment of the present invention, each of the first and the second spectrum sensors 221 and 223 may output a signal related to an intensity of light of a specific wavelength (e.g., 575 nm). The light that is emitted from each of the first and the second light sources 222 and 224 is transmitted through the control tube 140 or the test tube 130 and measured by a corresponding one of the first and the second spectrum sensors 221 and 223. That is, the intensity of the light, which is measured by each of the first and the second spectrum sensors 221 and 223, is an intensity of the light that is transmitted through the control tube 140 or the test tube 130. The intensity of the light may be converted into a current value by the first and the second spectrum sensors 221 and 223, and an analog current value may be converted into a digital binary value, and the digital binary value may be converted into 14 bits. Therefore, in the example illustrated in FIG 5, a unit indicated on the left vertical axis may be understood as a value indicating a relative intensity of the light that is transmitted through the control tube 140 or the test tube 130.

When the test starts, the heating unit 250 may heat the test tube 130 and the control tube 140 in response to a control signal output from the controller. The heating starts at 0 minutes, 0 to 2 minutes is a section in which the temperature of the heating unit 250 itself is increased, 2 to 15 minutes is a heating section for amplification, and 15 to 22 minutes is a section in which the heating is stopped and cooling is performed. In FIG 5, a line represented by circular marks indicates the internal temperature of the heating unit 250, a line represented by triangular marks indicates the absorbance of the control tube 140, and a line represented by square marks indicates a change in absorbance of the control tube 140.

In the heating section, since the temperatures of the control tube 140 and the test tube 130 themselves are increased and the color of the indicator may be changed, the absorbance may be increased. However, in the case of a negative sample, since there is no amplification in the test tube 130, the absorbance may be reduced when the temperature is lowered. On the other hand, in the control tube 140, even when amplification occurs and the temperature is lowered, the absorbance may be increased or maintained. That is, since the absorbance of the control tube 140 is greater than or equal to a preset measurement invalidity determination reference value, the test may be determined as a valid test, and since the absorbance of the test tube 130 is less than or equal to an infection determination reference value, a result of the test may be determined as "negative."

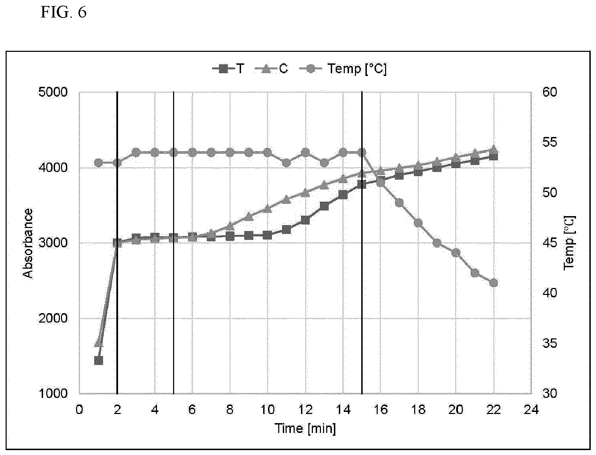

FIG. 6 illustrates an example of changes in absorbance over time of a control tube and a test tube with respect to a sample determined as positive.

Referring to FIG 6, horizontal/vertical axes and each item are the same as those in FIG 5, and thus redundant descriptions thereof will be omitted. Similarly, in a heating section of FIG 6, since the temperatures of the control tube 140 and the test tube 130 themselves are increased and the color of the indicator may be changed, the absorbance may be increased. However, in the case of a positive sample, since there is amplification in the test tube 130, the absorbance may be increased or maintained even when the temperature is lowered. Further, in the control tube 140, even when amplification occurs and the temperature is lowered, the absorbance may be increased or maintained. That is, since the absorbance of the control tube 140 is greater than or equal to a preset measurement invalidity determination reference value, the test may be determined as a valid test, and since the absorbance of the test tube 130 is greater than or equal to an infection determination reference value, a result of the test may be determined as "positive."

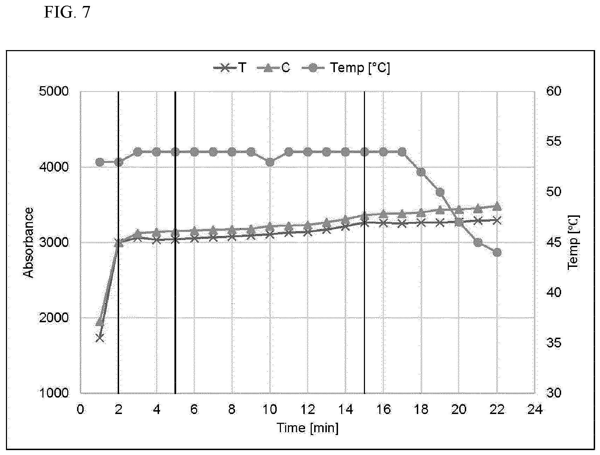

FIG 7 illustrates an example of changes in absorbance over time of a control tube and a test tube with respect to a sample, which is positive but collected in insufficient quantities.

Referring to FIG 7, it can be seen that a slope of a graph of absorbance of the control tube 140 is lower than that in each of the examples illustrated in FIGS. 5 and 6. The example illustrated in FIG 7 is an example in which, although the sample is positive, since the absorbance of the control tube 140 is greater than or equal to a preset measurement invalidity determination reference value, the test is determined as a valid test, and since the absorbance of the test tube 130 is less than or equal to an infection determination reference value, a result of the test is determined as "negative." That is, when a change in absorbance of the test tube 130 over time is qualitatively determined, the example illustrated in FIG 7 is the case in which, although it may be determined as positive, the result of the test is determined as negative due to the finally measured value of the absorbance. Therefore, there is a need for a method in which the controller 210 may determine the result of the test as positive or negative even when an amount of amplification is small.

The controller 210 according to the present invention may determine whether a sample is infected according to a ratio of change in absorbance of two tubes. The two tubes are the control tube 140 and the test tube 130, and the ratio of the change refers to a change in amount of amplification in the test tube compared to a change in amount of amplification in the control tube.

More specifically, the controller 210 may convert signals output from the first spectrum sensor 221 and signals output from the second spectrum sensor 223 at a first time point and a second time point and store the converted signals. The first time point and the second time point are preset time points, and the first time point and the second time point are different time points. However, the first spectrum sensor 221 and the second spectrum sensor 223 simultaneously output the signals at the first time point, and similarly, the first spectrum sensor 221 and the second spectrum sensor 223 simultaneously output the signals at the second time point. The first time point may be a time point relatively earlier than the second time point, and the first time point and the second time point may have an interval which is greater than or equal to a preset time.

According to an embodiment of the present invention, the first time point may be a time point adjacent to a time point at which the controller 210 terminates the heating operation of the heating unit 250. That is, the first time point may be a time point immediately before or immediately after the controller 210 terminates the heating operation of the heating unit 250. Further, the second time point may be a time point when a preset time has elapsed since the controller 210 terminated the heating operation of the heating unit 250. Preferably, the second time point may be a time point sufficiently far from the time point at which the heating operation is terminated. As another example, the second time point may be a time point when a temperature value output from the temperature sensor 251 reaches a preset temperature value after the controller 210 terminates the heating operation of the heating unit 250. Preferably, the preset temperature value is a value at which discoloration of the indicator is not affected by the temperature.

The controller 210 may calculate a difference value (hereinafter, referred to as a "control spectrum difference value") between a value of the signal output from the first spectrum sensor 221 at the first time point and a value of the signal output from the first spectrum sensor 221 at the second time point. In addition, the controller 210 may calculate a difference value (hereinafter, referred to as a "test spectrum difference value") between a value of the signal output from the second spectrum sensor 223 at the first time point and a value of the signal output from the second spectrum sensor 223 at the second time point. In addition, the controller 210 may determine whether the sample is infected using the test spectrum difference value compared to the control spectrum difference value.

More specifically, the controller 210 may determine whether the sample is infected using Equation 1 below.

<Equation 1>

$$K = (T_2 - T_1) / (C_2 - C_1)$$

- K denotes an infection determination value

- $T_2$ denotes a value output from the second spectrum sensor at the second time point

- $T_1$ denotes a value output from the second spectrum sensor at the first time point

- $C_2$ denotes a value output from the first spectrum sensor at the second time point

- Ci denotes a value output from the first spectrum sensor at the first time point

In this case, when the infection determination value K is greater than or equal to a preset reference value, the controller 210 may determine that a result of determining whether the sample is infected is positive. Hereinafter, Tables 1 and 2 are examples in which the result of determining whether the sample is infected is determined as negative and positive when the preset reference value is "0."

<Table 1>

| Sample type | $T_2$-$T_1$ | $C_2$-$C_1$ | K | Test reagent photograph | Control reagent photograph |
|---|---|---|---|---|---|
| Negative | -102.5 | 177 | -0.5791 | | |
| Positive #1 | 305 | 176 | 1.6550 | | |
| Positive #2 | 84.3 | 57 | 1.4789 | | |

<Table 2>

| Sample type | $T_2$-$T_1$ | $C_2$-$C_1$ | K | Test reagent photograph | Control reagent photograph |
|---|---|---|---|---|---|
| Negative | 157.8 | 162 | -0.3568 | | |
| Positive #1 | 424 | 243 | 1.4260 | | |

Meanwhile, when the value of the signal output from the first spectrum sensor 221 at the second time point is greater than or equal to a preset measurement invalidity determination reference value and when the value of the signal output from the second spectrum sensor 223 at the second time point is less than or equal to a preset primary infection determination reference value, the controller 210 according to the present invention may determine whether the sample is infected using the test spectrum difference value compared to the control spectrum difference value. In other words, when the primary determination is "negative," whether the sample is infected may be secondarily determined by supplementarily using the test spectrum difference value compared to the control spectrum difference value.

Thereafter, the communication unit 230 may transmit the value of the signal output from the first spectrum sensor, the value of the signal output from the second spectrum sensor, or information on whether the sample is infected as determined by the controller to the external terminal 300. In this case, the external terminal 300 may receive the data transmitted from the test apparatus and display the information on whether the sample is infected on a display.

The embodiment in which the test apparatus 200 itself determines whether the sample is infected has been described. The diagnostic system 10 according to the present invention includes not only the test apparatus 200 but also the external terminal 300, and thus the test apparatus 200 may provide measurement values and the external terminal 300 may determine whether the sample is infected. Hereinafter, a test method according to the present invention will be described on the premise of a computer program executed in the external terminal 300 to which the measurement values are provided. However, the test method described below is not necessarily limited to being performed in the external terminal 300 such as a smartphone, and may be performed in the test apparatus 200.

The external terminal 300 may include a communication unit that receives the value of the signal output from the first spectrum sensor 221 and the value of the signal output from the second spectrum sensor 223 from the test apparatus 200, and a processor that performs each of operations of the test method which will be described below.

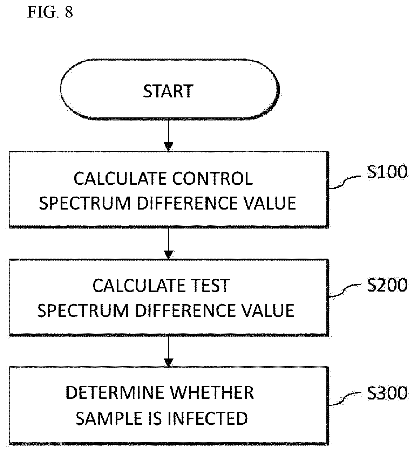

FIG 8 is a flowchart of a test method according to an embodiment of the present invention.

Referring to FIG 8, in operation S100, a processor may calculate a difference value (hereinafter, referred to as a "control spectrum difference value") between a value of a signal output from a first spectrum sensor that outputs a signal related to a wavelength of light emitted from a first light source and transmitted through a control tube at a preset first time point, and a value of a signal output from the first spectrum sensor at a preset second time point different from the first time point. The first time point may be a time point relatively earlier than the second time point. Further, the first time point and the second time point may have an interval which is greater than or equal to a preset time.

Next, in operation S200, the processor may calculate a difference value (hereinafter, referred to as a "test spectrum difference value") between a value of a signal output from a second spectrum sensor that outputs a signal related to a wavelength of light emitted from a second light source and transmitted through a test tube at the first time point, and a value of a signal output from the second spectrum sensor at the second time point.

Next, in operation S300, the processor may determine whether a sample is infected using the test spectrum difference value compared to the control spectrum difference value. In operation S300, the processor may determine whether the sample is infected using Equation 1 described above. In addition, when the infection determination value K is greater than or equal to the preset reference value, the processor may determine that a result of determining whether the sample is infected is positive.

Meanwhile, operation S300 may be performed when the value of the signal output from the first spectrum sensor at the second time point is greater than or equal to a preset measurement invalidity determination reference value and when the value of the signal output from the second spectrum sensor at the second time point is less than or equal to a preset primary infection determination reference value.

The controller 210 may include a processor, an application-specific integrated circuit (ASIC), another chipset, a logic circuit, a register, a communication modem, a data processing device, and the like, which are known in the art in order to execute calculations and various control logics. Further, when the above-described control logic is implemented in software, the controller may be implemented as a set of program modules. In this case, the program modules may be stored in a memory and may be performed by the processor.

The computer program may include code coded in a computer language such as C/C++, C#, JAVA, Python, or machine language that a processor (i.e., a central processing processor (CPU)) of the computer may read through a device interface of the computer in order for the computer to read the program and execute the methods implemented as a program. The code may include functional code related to a function defining functions necessary to execute the above methods and the like and include execution procedure related control code necessary for the processor of the computer to execute the functions according to a predetermined procedure. Further, the code may further include memory reference-related code for referencing a location (address) of an internal or external memory of the computer where there is additional information or media necessary for the processor of the computer to execute the functions. Further, when the processor of the computer needs to communicate with any other computer or server in a remote location in order to execute the functions, the code may further include communication-related code for how to communicate with any other remote computer or server using the communication module of the computer and what information or media to transmit or receive during communication.

The storage medium is not a medium that stores data for a short moment, such as a register, a cache, a memory, etc., but a medium that stores data semi-permanently and may be read by a device. Specifically, examples of the storage medium include a read only memory (ROM), a random access memory (RAM), a compact disc read only memory (CD-ROM), a magnetic tape, a floppy disk, an optical data storage device, and the like, but the present invention is not limited thereto. That is, the program may be stored in various recording media on various servers accessible by the computer or in various recording media on the computer of the user. Further, the media may be distributed to computer systems connected by a network, and computer-readable code may be stored in a distributed manner.

According to one aspect of the present invention, even when an amount of sample collected is small, it is possible to determine whether an infection is present.

According to another aspect of the present invention, even when an amount of amplification is small due to an external environment, it is possible to determine whether an infection is present.

Effects of the present invention are not limited to the above-described effects and other effects that are not described will be clearly understood by those skilled in the art from the above detailed descriptions.

While embodiments of the present inventive concept have been described with reference to the accompanying drawings, it will be understood by those skilled in the art that various modifications can be made without departing from the scope of the present inventive concept and without changing essential features. Therefore, the above-described embodiments should be considered in a descriptive sense only and not for purposes of limitation.

**Claims**

1. A test apparatus (200) comprising:

   a first spectral sensor (221) configured to output a signal related to a wavelength of light emitted from a first light source and transmitted through a control tube (140);
   a second spectral sensor (223) configured to output a signal related to the at least one wavelength of light emitted from a second light source and transmitted through a test tube (130); and
   a controller (210) configured to determine whether a sample is infected using a test spectral difference value between a value of a signal output from the second spectral sensor (223) at a preset first time point and a value of a signal output from the second spectral sensor (223) at a preset second time point different from the first time point compared to a control spectral difference value between a value of a signal output from the first spectral sensor (221) at the first time point and a value of a signal output from the first spectral sensor (221) at the second time point.

2. The test apparatus of claim 1, further comprising a heating unit (250) configured to heat the test tube (130) and the control tube (140) according to a control signal output from the controller (210).

3. The test apparatus of claim 2, wherein:

   the first time point is a time point adjacent to a time point at which the controller (210) terminates heating operation of the heating unit (250); and
   the second time point is a time point when a preset time has elapsed since the controller (210) terminated the heating operation of the heating unit (250).

4. The test apparatus of claim 2 or 3, further comprising a temperature sensor (251) configured to output a temperature value of the control tube (140), the test tube (130), or the heating unit (250),
   wherein the second time point is a time point when the temperature value output from the temperature sensor (251) reaches a preset temperature value after the controller (210) terminates heating operation of the heating unit (250).

5. The test apparatus of one of claims 1 to 4, wherein the controller (210) determines whether the sample is infected using the following equation:

$$K = (T_2 - T_1) / (C_2 - C_1)$$

   - K denotes an infection determination value,
   - $T_2$ denotes a value output from the second spectrum sensor (223) at the second time point,
   - $T_1$ denotes a value output from the second spectrum sensor (223) at the first time point,
   - $C_2$ denotes a value output from the first spectrum sensor (221) at the second time point, and
   - Ci denotes a value output from the first spectrum sensor (221) at the first time point.

6. The test apparatus of claim 5, wherein, when the infection determination value (K) is greater than or equal to a preset reference value, the controller (210) determines that a result of determining whether the sample is infected is positive.

7. The test apparatus of one of claims 1 to 6, wherein, when the value of the signal output from the second spectrum sensor (223) at the second time point is less than or equal to a preset primary infection determination reference value, the controller (210) determines whether the sample is infected using the test spectrum difference value compared to the control spectrum difference value.

8. The test apparatus of one of claims 1 to 7, wherein, when the value of the signal output from the first spectrum sensor (221) at the second time point is greater than or equal to a preset measurement invalidity determination reference value and when the value of the signal output from the second spectrum sensor (223) at the second time point is less than or equal to a preset primary infection determination reference value, the controller (210) determines whether the sample is infected using the test spectrum difference value compared to the control spectrum difference value.

9. The test apparatus of one of claims 1 to 8, further comprising a communication unit (230) configured to transmit the value of the signal output from the first spectrum sensor (221), the value of the signal output from the second spectrum sensor (223), or information on whether the sample is infected as determined by the controller (210).

10. A diagnostic system comprising:

   the test apparatus according to claim 1; and
   an external terminal configured to receive data transmitted from the test apparatus and display information on whether a sample is infected.

11. A test method comprising:

   an operation (a) of calculating, by a processor, a control spectral difference value between a value of a signal output from a first spectral sensor (221) that outputs a signal related to a wavelength of light emitted from a first light source and transmitted through a control tube (140) at a preset first time point, and a value of a signal output from the first spectral sensor (221) at a preset second time point different from the first time point;
   an operation (b) of calculating, by the processor, a test spectral difference value between a value of a signal output from a second spectral sensor (223) that outputs a signal related to the at least one wavelength of light emitted from a second light source and transmitted through a test tube (130) at the first time point, and a value of a signal output from the second spectral sensor (223) at the second time point; and
   an operation (c) of determining, by the processor, whether a sample is infected using the test spectral difference value compared to the control spectral difference value.

12. The test method of claim 11, wherein the first time point is a time point relatively earlier than the second time point.

13. The test method of claim 12, wherein the first time point and the second time point have an interval which is greater than or equal to a preset time.

14. The test method of one of claims 11 to 13, wherein the operation (c) includes an operation of determining, by the processor, whether the sample is infected using the following equation:

$$K = (T_2 - T_1) / (C_2 - C_1)$$

   - K denotes an infection determination value,
   - $T_2$ denotes a value output from the second spectrum sensor (223) at the second time point,
   - $T_1$ denotes a value output from the second spectrum sensor (223) at the first time point,
   - $C_2$ denotes a value output from the first spectrum sensor (221) at the second time point, and
   - Ci denotes a value output from the first spectrum sensor (221) at the first time point.

15. The test method of claim 14, wherein the operation (c) includes, when the infection determination value (K) is greater than or equal to a preset reference value, an operation of determining, by the processor, that a result of determining whether the sample is infected is positive.

16. The test method of one of claims 11 to 15, wherein the operation (c) includes, when the value of the signal output from the second spectrum sensor (223) at the second time point is less than or equal to a preset primary infection determination reference value, an operation of determining, by the processor, whether the sample is infected using the test spectrum difference value compared to the control spectrum difference value.

17. The test method of one of claims 11 to 16, wherein the operation (c) includes, when the value of the signal output from the first spectrum sensor (221) at the second time point is greater than or equal to a preset measurement invalidity determination reference value, and when the value of the signal output from the second spectrum sensor (223) at the second time point is less than or equal to a preset primary infection determination reference value, an operation of determining, by the processor, whether the sample is infected using the test spectrum difference value compared to the control spectrum difference value.

18. A computer program written to cause the test apparatus of any of claims 1-10 to perform each of the operations of the test method according to one of claims 11 to 17 on a computer and recorded on a computer-readable recording medium.

19. A terminal (261) comprising:

a processor configured to cause the test apparatus of any of claims 1-10 to perform each of the operations of the test method according to one of claims 11 to 17; and
a communication unit (230) configured to receive, from a test apparatus (200), a value of a signal output from the first spectrum sensor (221) and a value of a signal output from the second spectrum sensor (223).

20. A diagnostic system (10) comprising:

the terminal according to claim 19; and
a test apparatus (200) including the first spectrum sensor (221) and the second spectrum sensor (223).

**Patentansprüche**

1. Testvorrichtung (200), umfassend:

einen ersten Spektralsensor (221), der dazu ausgebildet ist, ein Signal bezogen auf eine Wellenlänge von Licht, das von einer ersten Lichtquelle emittiert wird und durch ein Kontrollröhrchen (140) übertragen wird, auszugeben;
einen zweiten Spektralsensor (223), der dazu ausgebildet ist, ein Signal bezogen auf die mindestens eine Wellenlänge von Licht, das von einer zweiten Lichtquelle emittiert wird und durch ein Teströhrchen (130) übertragen wird, auszugeben; und
eine Steuerung (210), die dazu ausgebildet ist, unter Verwendung eines Testspektraldifferenzwerts zwischen einem Wert eines Signals, das von dem zweiten Spektralsensor (223) zu einem voreingestellten ersten Zeitpunkt ausgegeben wird, und einem Wert eines Signals, das von dem zweiten Spektralsensor (223) zu einem voreingestellten zweiten Zeitpunkt ausgegeben wird, der anders ist als der erste Zeitpunkt, verglichen mit einem Kontroll spektral differenzwert zwischen einem Wert eines Signals, das von dem ersten Spektralsensor (221) zu dem ersten Zeitpunkt ausgegeben wird, und einem Wert eines Signals, das von dem ersten Spektralsensor (221) zu dem zweiten Zeitpunkt ausgegeben wird, zu bestimmen, ob eine Probe infiziert ist.

2. Testvorrichtung nach Anspruch 1, des Weiteren umfassend eine Heizeinheit (250), die dazu ausgebildet ist, das Teströhrchen (130) und das Kontrollröhrchen (140) gemäß einem von der Steuerung (210) ausgegebenen Steuersignal zu erhitzen.

3. Testvorrichtung nach Anspruch 2, wobei:

der erste Zeitpunkt ein Zeitpunkt ist, der an einen Zeitpunkt angrenzt, zu dem die Steuerung (210) den Heizvorgang der Heizeinheit (250) abschließt; und
der zweite Zeitpunkt ein Zeitpunkt ist, wenn eine voreingestellte Zeit verstrichen ist, seit die Steuerung (210) den Heizvorgang der Heizeinheit (250) abgeschlossen hat.

4. Testvorrichtung nach Anspruch 2 oder 3, des Weiteren umfassend einen Temperatursensor (251), der dazu ausgebildet ist, einen Temperaturwert des Kontrollröhrchens (140), des Teströhrchens (130) oder der Heizeinheit (250) auszugeben,
wobei der zweite Zeitpunkt ein Zeitpunkt ist, wenn der von dem Temperatursensor (251) ausgegebene Temperaturwert, nachdem die Steuerung (210) den Heizvorgang der Heizeinheit (250) abgeschlossen hat, einen voreingestellten Temperaturwert errei cht.

5. Testvorrichtung nach einem der Ansprüche 1 bis 4, wobei die Steuerung (210) bestimmt, ob die Probe infiziert ist, indem sie die folgende Gleichung verwendet:

$$K = (T_2 - T_1) / (C_2 - C_1)$$

- K gibt einen Infektionsbestimmungswert an,
- $T_2$ gibt einen von dem zweiten Spektrumssensor (223) zu dem zweiten Zeitpunkt ausgegebenen Wert an,
- $T_1$ gibt einen von dem zweiten Spektrumssensor (223) zu dem ersten Zeitpunkt ausgegebenen Wert an,
- $C_2$ gibt einen von dem ersten Spektrumssensor (221) zu dem zweiten Zeitpunkt ausgegebenen Wert an, und
- Ci gibt einen von dem ersten Spektrumssensor (221) zu dem ersten Zeitpunkt ausgegebenen Wert an.

6. Testvorrichtung nach Anspruch 5, wobei, wenn der Infektionsbestimmungswert (K) größer als ein voreingestellter oder gleich einem voreingestellten Referenzwert ist, die Steuerung (210) bestimmt, dass ein Ergebnis der Bestimmung, ob die Probe infiziert ist, positiv ist.

7. Testvorrichtung nach einem der Ansprüche 1 bis 6, wobei, wenn der Wert des Signals, das von dem zweiten Spektrumssensor (223) zu dem zweiten Zeitpunkt ausgegeben wird, kleiner als ein voreingestellter primärer oder gleich einem voreingestellten primären Infektionsbestimmungsreferenzwert ist, die Steuerung (210) bestimmt, ob die Probe infiziert ist, indem sie den Testspektrumsdifferenzwert verglichen mit dem Kontrollspektrumsdifferenzwert verwendet.

8. Testvorrichtung nach einem der Ansprüche 1 bis 7, wobei, wenn der Wert des Signals, das von dem ersten Spektrumssensor (221) zu dem zweiten Zeitpunkt ausgegeben wird, größer als ein voreingestellter oder gleich einem voreingestellten Messungültigkeitsbestimmungsreferenzwert ist und wenn der Wert des Signals, das von dem zweiten Spektrumssensor (223) zu dem zweiten Zeitpunkt ausgegeben wird, kleiner als ein voreingestellter primärer oder gleich einem voreingestellten primären Infektionsbestimmungsreferenzwert ist, die Steuerung (210) bestimmt, ob die Probe infiziert ist, indem sie den Testspektrumsdifferenzwert verglichen mit dem Kontrollspektrumsdifferenzwert verwendet.

9. Testvorrichtung nach einem der Ansprüche 1 bis 8, des Weiteren umfassend eine Kommunikationseinheit (230), die dazu ausgebildet ist, den Wert des Signals, das von dem ersten Spektrumssensor (221) ausgegeben wird, den Wert des Signals, das von dem zweiten Spektrumssensor (223) ausgegeben wird, oder Informationen darüber, ob die Probe, wie durch die Steuerung (210) bestimmt, infiziert ist, zu übertragen.

10. Diagnosesystem, umfassend:

    die Testvorrichtung nach Anspruch 1; und
    ein externes Endgerät, das dazu ausgebildet ist, Daten zu empfangen, die von der Testvorrichtung übertragen werden, und Informationen darüber anzuzeigen, ob eine Probe infiziert ist.

11. Testverfahren, umfassend:

    einen Vorgang (a) des Berechnens durch einen Prozessor eines Kontrollspektraldifferenzwerts zwischen einem Wert eines Signals, das von einem ersten Spektralsensor (221) ausgegeben wird, der zu einem voreingestellten ersten Zeitpunkt ein Signal ausgibt bezogen auf eine Wellenlänge von Licht, das von einer ersten Lichtquelle emittiert wird und durch ein Kontrollröhrchen (140) übertragen wird, und einem Wert eines Signals, das von dem ersten Spektralsensor (221) zu einem voreingestellten zweiten Zeitpunkt ausgegeben wird, der anders ist als der erste Zeitpunkt;
    einen Vorgang (b) des Berechnens durch den Prozessor eines Testspektraldifferenzwerts zwischen einem Wert eines Signals, das von einem zweiten Spektralsensor (223) ausgegeben wird, der zu dem ersten Zeitpunkt ein Signal ausgibt bezogen auf die mindestens eine Wellenlänge von Licht, das von einer zweiten Lichtquelle emittiert wird und durch ein Teströhrchen (130) übertragen wird, und einem Wert eines Signals, das von dem zweiten Spektralsensor (223) zu einem zweiten Zeitpunkt ausgegeben wird; und
    einen Vorgang (c) des Bestimmens durch den Prozessor, ob eine Probe infiziert ist, indem der Testspektraldifferenzwert verglichen mit dem Kontrollspektraldifferenzwert verwendet wird.

12. Testverfahren nach Anspruch 11, wobei der erste Zeitpunkt ein Zeitpunkt ist, der relativ früher als der zweite Zeitpunkt ist.

**13.** Testverfahren nach Anspruch 12, wobei der erste Zeitpunkt und der zweite Zeitpunkt ein Intervall aufweisen, das größer als eine voreingestellte oder gleich einer voreingestellten Zeit ist.

**14.** Testverfahren nach einem der Ansprüche 11 bis 13, wobei der Vorgang (c) einen Vorgang des Bestimmens durch den Prozessor umfasst, ob die Probe infiziert ist, indem folgende Gleichung verwendet wird:

$$K = (T_2 - T_1) / (C_2 - C_1)$$

- K gibt einen Infektionsbestimmungswert an,
- $T_2$ gibt einen von dem zweiten Spektrumssensor (223) zu dem zweiten Zeitpunkt ausgegebenen Wert an,
- $T_1$ gibt einen von dem zweiten Spektrumssensor (223) zu dem ersten Zeitpunkt ausgegebenen Wert an,
- $C_2$ gibt einen von dem ersten Spektrumssensor (221) zu dem zweiten Zeitpunkt ausgegebenen Wert an, und
- Ci gibt einen von dem ersten Spektrumssensor (221) zu dem ersten Zeitpunkt ausgegebenen Wert an.

**15.** Testverfahren nach Anspruch 14, wobei der Vorgang (c), wenn der Infektionsbestimmungswert (K) größer als ein voreingestellter oder gleich einem voreingestellten Referenzwert ist, einen Vorgang des Bestimmens durch den Prozessor umfasst, dass ein Ergebnis der Bestimmung, ob die Probe infiziert ist, positiv ist.

**16.** Testverfahren nach einem der Ansprüche 11 bis 15, wobei der Vorgang (c), wenn der Wert des Signals, das von dem zweiten Spektrumssensor (223) zu dem zweiten Zeitpunkt ausgegeben wird, kleiner als ein voreingestellter primärer oder gleich einem voreingestellten primären Infektionsbestimmungsreferenzwert ist, einen Vorgang des Bestimmens durch den Prozessor umfasst, ob die Probe infiziert ist, indem der Testspektrumsdifferenzwert verglichen mit dem Kontrollspektrumsdifferenzwert verwendet wird.

**17.** Testverfahren nach einem der Ansprüche 11 bis 16, wobei der Vorgang (c), wenn der Wert des Signals, das von dem ersten Spektrumssensor (221) zu dem zweiten Zeitpunkt ausgegeben wird, größer als ein voreingestellter oder gleich einem voreingestellten Messungültigkeitsbestimmungsreferenzwert ist und wenn der Wert des Signals, das von dem zweiten Spektrumssensor (223) zu dem zweiten Zeitpunkt ausgegeben wird, kleiner als ein voreingestellter primärer oder gleich einem voreingestellten primären Infektionsbestimmungsreferenzwert ist, einen Vorgang des Bestimmens durch den Prozessor umfasst, ob die Probe infiziert ist, indem der Testspektrumsdifferenzwert verglichen mit dem Kontrollspektrumsdifferenzwert verwendet wird.

**18.** Computerprogramm, das dazu geschrieben ist, dass es bewirkt, dass die Testvorrichtung nach einem der Ansprüche 1 bis 10 jeden der Vorgänge des Testverfahrens nach einem der Ansprüche 11 bis 17 auf einem Computer durchführt, und das auf einem computerlesbaren Aufzeichnungsmedium aufgezeichnet ist.

**19.** Endgerät (261), umfassend:

einen Prozessor, der dazu ausgebildet ist, zu bewirken, dass die Testvorrichtung nach einem der Ansprüche 1 bis 10 jeden der Vorgänge des Testverfahrens nach einem der Ansprüche 11 bis 17 durchführt; und
eine Kommunikationseinheit (230), die dazu ausgebildet ist, von einer Testvorrichtung (200) einen Wert eines Signals, das von dem ersten Spektrumssensor (221) ausgegeben wird, und einen Wert eines Signals, das von dem zweiten Spektrumssensor (223) ausgegeben wird, zu empfangen.

**20.** Diagnosesystem (10), umfassend:

das Endgerät nach Anspruch 19; und
eine Testvorrichtung (200), umfassend den ersten Spektrumssensor (221) und den zweiten Spektrumssensor (223).

**Revendications**

**1.** Appareil d'essai (200), comprenant:

un premier capteur spectral (221) configuré pour émettre un signal lié à une longueur d'onde de lumière émise à partir d'une première source de lumière et transmise à travers un tube de contrôle (140) ;

un deuxième capteur spectral (223) configuré pour émettre un signal lié à l'au moins une longueur d'onde de lumière émise à partir d'une deuxième source de lumière et transmise à travers un tube d'essai (130) ; et un dispositif de contrôle (210) configuré pour déterminer si un échantillon est infecté en utilisant une valeur de différence spectrale d'essai entre une valeur d'un signal émis à partir du deuxième capteur spectral (223) à un premier moment préréglé et une valeur d'un signal émis à partir du deuxième capteur spectral (223) à un deuxième moment préréglé qui est différent du premier moment comparée à une valeur de différence spectrale de contrôle entre une valeur d'un signal émis à partir du premier capteur spectral (221) au premier moment et une valeur d'un signal émis à partir du premier capteur spectral (221) au deuxième moment.

2. Appareil d'essai selon la revendication 1, comprenant en outre une unité de chauffage (250) configurée pour chauffer le tube d'essai (130) et le tube de contrôle (140) selon un signal de contrôle émis à partir du dispositif de contrôle (210).

3. Appareil d'essai selon la revendication 2, dans lequel:

le premier moment est un moment adjacent à un moment auquel le dispositif de contrôle (210) termine l'opération de chauffage de l'unité de chauffage (250) ; et
le deuxième moment est un moment lorsqu'un temps préréglé s'est écoulé depuis que le dispositif de contrôle (210) a terminé l'opération de chauffage de l'unité de chauffage (250).

4. Appareil d'essai selon la revendication 2 ou 3, comprenant en outre un capteur de température (251) configuré pour émettre une valeur de température du tube de contrôle (140), du tube d'essai (130) ou de l'unité de chauffage (250), dans lequel le deuxième moment est un moment lorsque la valeur de température émise à partir du capteur de température (251) atteint une valeur de température préréglée après que le dispositif de contrôle (210) a terminé une opération de chauffage de l'unité de chauffage (250).

5. Appareil d'essai selon l'une quelconque des revendications 1 à 4, dans lequel le dispositif de contrôle (210) détermine si l'échantillon est infecté en utilisant l'équation suivante :

$$K = (T_2 - T_1) / (C_2 - C_1)$$

- K désigne une valeur de détermination de l'infection,
- $T_2$ désigne une valeur émise à partir du deuxième capteur de spectre (223) au deuxième moment,
- $T_1$ désigne une valeur émise à partir du deuxième capteur de spectre (223) au premier moment
- $C_2$ désigne une valeur émise à partir du premier capteur de spectre (221) au deuxième moment, et
- Ci désigne une valeur émise à partir du premier capteur de spectre (221) au premier moment.

6. Appareil d'essai selon la revendication 5, dans lequel, lorsque la valeur (K) de détermination de l'infection est supérieure ou égale à une valeur de référence préréglée, le dispositif de contrôle (210) détermine qu'un résultat de la détermination si l'échantillon est infecté est positif.

7. Appareil d'essai selon l'une quelconque des revendications 1 à 6, dans lequel, lorsque la valeur du signal émis à partir du deuxième capteur de spectre (223) au deuxième moment est inférieure ou égale à une valeur de référence de détermination de l'infection primaire préréglée, le dispositif de contrôle (210) détermine si l'échantillon est infecté en utilisant la valeur de différence de spectre d'essai comparée à la valeur de différence de spectre de contrôle.

8. Appareil d'essai selon l'une quelconque des revendications 1 à 7, dans lequel, lorsque la valeur du signal émis à partir du premier capteur de spectre (221) au deuxième moment est supérieure ou égale à une valeur de référence de détermination de l'invalidité de mesure préréglée et lorsque la valeur du signal émis à partir du deuxième capteur de spectre (223) au deuxième moment est inférieure ou égale à une valeur de référence de détermination de l'infection primaire préréglée, le dispositif de contrôle (210) détermine si l'échantillon est infecté en utilisant la valeur de différence de spectre d'essai comparée à la valeur de différence de spectre de contrôle.

9. Appareil d'essai selon l'une quelconque des revendications 1 à 8, comprenant en outre une unité de communication (230) configurée pour transmettre la valeur du signal émis à partir du premier capteur de spectre (221), la valeur du signal émis à partir du deuxième capteur de spectre (223) ou des informations indiquant si l'échantillon est infecté comme déterminé par le dispositif de contrôle (210).

**10.** Système de diagnostic, comprenant:

l'appareil d'essai selon la revendication 1; et
un terminal externe configuré pour recevoir des données transmises à partir de l'appareil d'essai et pour afficher des informations indiquant si un échantillon est infecté.

**11.** Procédé d'essai, comprenant:

une opération (a) de calculer par un processeur une valeur de différence spectrale de contrôle entre une valeur d'un signal émis à partir d'un premier capteur spectral (221) qui émet un signal lié à une longueur d'onde de lumière émise à partir d'une première source de lumière et transmise à travers un tube de contrôle (140) à un premier moment préréglé et une valeur d'un signal émis à partir du premier capteur spectral (221) à un deuxième moment préréglé qui est différent du premier moment;
une opération (b) de calculer par le processeur une valeur de différence spectrale d'essai entre une valeur d'un signal émis à partir d'un deuxième capteur spectral (223) qui émet un signal lié à l'au moins une longueur d'onde de lumière émise à partir d'une deuxième source de lumière et transmise à travers un tube d'essai (130) au premier moment et une valeur d'un signal émis à partir du deuxième capteur spectral (223) à un deuxième moment ; et
une opération (c) de déterminer par le processeur si un échantillon est infecté en utilisant la valeur de différence spectrale d'essai comparée à la valeur de différence spectrale de contrôle.

**12.** Procédé d'essai selon la revendication 11, dans lequel le premier moment est un moment relativement antérieur au deuxième moment.

**13.** Procédé d'essai selon la revendication 12, dans lequel le premier moment et le deuxième moment ont un intervalle qui est supérieur ou égal à un temps préréglé.

**14.** Procédé d'essai selon l'une quelconque des revendications 11 à 13, dans lequel l'opération (c) comprend une opération de déterminer par le processeur si l'échantillon est infecté en utilisant l'équation suivante:

$$K = (T_2 - T_1) / (C_2 - C_1)$$

- K désigne une valeur de détermination de l'infection,
- $T_2$ désigne une valeur émise à partir du deuxième capteur de spectre (223) au deuxième moment,
- $T_1$ désigne une valeur émise à partir du deuxième capteur de spectre (223) au premier moment
- $C_2$ désigne une valeur émise à partir du premier capteur de spectre (221) au deuxième moment, et
- Ci désigne une valeur émise à partir du premier capteur de spectre (221) au premier moment.

**15.** Procédé d'essai selon la revendication 14, dans lequel l'opération (c) comprend, lorsque la valeur (K) de détermination de l'infection est supérieure ou égale à une valeur de référence préréglée, une opération de déterminer par le processeur qu'un résultat de la détermination si l'échantillon est infecté est positif.

**16.** Procédé d'essai selon l'une quelconque des revendications 11 à 15, dans lequel l'opération (c) comprend, lorsque la valeur du signal émis à partir du deuxième capteur de spectre (223) au deuxième moment est inférieure ou égale à une valeur de référence de détermination de l'infection primaire préréglée, une opération de déterminer par le processeur si l'échantillon est infecté en utilisant la valeur de différence de spectre d'essai comparée à la valeur de différence de spectre de contrôle.

**17.** Procédé d'essai selon l'une quelconque des revendications 11 à 16, dans lequel l'opération (c) comprend, lorsque la valeur du signal émis à partir du premier capteur de spectre (221) au deuxième moment est supérieure ou égale à une valeur de référence de détermination de l'invalidité de mesure préréglée et lorsque la valeur du signal émis à partir du deuxième capteur de spectre (223) au deuxième moment est inférieure ou égale à une valeur de référence de détermination de l'infection primaire préréglée, une opération de déterminer par le processeur si l'échantillon est infecté en utilisant la valeur de différence de spectre d'essai comparée à la valeur de différence de spectre de contrôle.

**18.** Programme d'ordinateur écrit pour amener l'appareil d'essai selon l'une quelconque des revendications 1 à 10 à effectuer chacune des opérations du procédé d'essai selon l'une quelconque des revendications 11 à 17 sur un ordinateur et enregistré sur un support d'enregistrement lisible par ordinateur.

19. Terminal (261), comprenant:

un processeur configuré pour amener l'appareil d'essai selon l'une quelconque des revendications 1 à 10 à effectuer chacune des opérations du procédé d'essai selon l'une quelconque des revendications 11 à 17 ; et une unité de communication (230) configurée pour recevoir à partir d'un appareil d'essai (200) une valeur d'un signal émis à partir du premier capteur de spectre (221) et une valeur d'un signal émis à partir du deuxième capteur de spectre (223).

20. Système de diagnostic (10), comprenant:

le terminal selon la revendication 19 ; et
un appareil d'essai (200) comprenant le premier capteur de spectre (221) et le deuxième capteur de spectre (223).

EP 4 215 903 B1

FIG. 1

19

FIG. 2

**200**

FIG. 3

(a)                              (b)

FIG. 4

| DETERMINATION RESULT | | RESULT INTERPRETATION |
| --- | --- | --- |
| Test tube (SARS-CoV-2) | Control tube (IC) | |
| Positive (YELLOW) | Positive (YELLOW) | SARS-CoV-2    VIRUS IS DETECTED |
| Negative (RED) | Positive (YELLOW) | SARS-CoV-2    VIRUS IS NOT DETECTED |
| Positive (YELLOW) | Negative (RED) | TEST IS INVALID: RETEST IS RECOMMENDED |
| Negative (RED) | Negative (RED) | |

22

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20220009053

- US 20180016627 A1

**Non-patent literature cited in the description**

- **JACK FERGUSON ; STEVEN DUNN ; ANGUS BEST.** Validation testing to determine the sensitivity of lateral flow testing for asymptomatic SARS-CoV-2 detection in low prevalence settings: Testing frequency and public health messaging is key. *PLOS Biology,* 29 April 2021

- **CHANG, WEN-HSIN et al.** Rapid isolation and detection of aquaculture pathogens in an integrated microfluidic system using loop-mediated isothermal amplification. *Sensors and Actuators B: Chemical,* 2013, vol. 180, 96-106